# EUROPÄISCHE PATENTSCHRIFT

(11) **EP 0 917 864 B1**
(45) Veröffentlichungstag und Bekanntmachung des Hinweises auf die Patenterteilung: **29.08.2007**
(21) Anmeldenummer: 98120301.1
(22) Anmeldetag: 27.10.1998
(51) Int. Cl.: A61F 5/02

(54) **Minimalorthese zur Behandlung der Osteoporose**
Minimal orthesis for the treatment of osteoporosis
Orthèse minimale pour le traitement de l'osteoporose

(30) Priorität: 19.11.1997 DE 29720475 U
(43) Veröffentlichungstag der Anmeldung: 26.05.1999
(73) Patentinhaber: Medi Bayreuth Weihermüller & Voigtmann GmbH & Co. KG, 95448 Nürnberg (DE)
(72) Erfinder: Minne, Helmut W., Prof. Dr. med., 31812 Bad Pyrmont (DE)
(74) Vertreter: Schuhmann, Albrecht

(56) Entgegenhaltungen:
- DE-A- 3 232 638
- GB-A- 1 024 686
- GB-A- 1 080 241
- US-A- 1 581 791
- US-A- 2 828 737
- US-A- 4 261 349
- US-A- 5 619 747

## Beschreibung

Die Erfindung betrifft eine Orthese zur Behandlung der Osteoporose mit den Merkmalen des Oberbegriffs des Anspruchs 1.

Es sind verschiedene Arten von Orthesen zur Behandlung der Osteoporose bekannt. So zeigt die DE-C-39 28 628, die unter dem Namen medi 3C vertrieben wird, eine Hyperextensionsorthese mit einer Basisplatte, einem sich davon nach unten erstreckenden Bauchstab mit Symphysenpelotte, mit sich nach beiden Seiten erstreckenden Stegen mit Verschlusselementen und einem sich nach oben erstreckenden Bruststab an dem verschiebbar eine Sternalpelotte angeordnet ist. Eine andere Hyperextensionsorthese, die unter dem Namen medi 4C vertrieben wird, verzichtet auf Bauchstab und Bruststab und erzielt eine axiale Stabilisierung der Brust- und Lendenwirbelsäule durch eine stabile Rahmenbauweise, wobei der Rumpf unterhalb des Schlüsselbeins gegen Vorwärtsneigung abgestützt ist. Ein orthopädisches Stützkorsett zeigt die DE-A-27 51 608, bei dem die Wirbelsäule elastisch abgestützt werden soll, wobei eine Schiene aus elastischen Gliedern an der Wirbelsäule anliegt und durch Befestigungsmittel an den Schultern und Oberschenkeln fixiert ist. Die US-A-2 828 737 offenbart eine Orthese mit einer die Wirbelsäule abstützenden steifen Schiene, sowie über den Rumpf geführte Befestigungsmittel und obere Befestigungsgurte, die nach Art von Rucksackträgern über die Schultern und zurück unter den Achseln hindurch geführt sind und beiderseits in Höhe des Schulterblattes auf der Rückseite befestigt sind, die die Brustatmung nicht behindern soll.
Der Einbruch eines Wirbelkörpers ist eine typische Spätkomplikation der Osteoporose. Die entstehende Deformierung des Wirbelkörpers führt zu Fehlstellungen der Zwischenwirbelgelenke, Fehlfunktionen der Sehnen, Bänder und Muskulatur. Dies wird zur Ursache für chronischen Schmerz, für Behinderung im Alltag.

Aufgabe einer Orthese ist es dann, die Wirbelsäule wieder aufzurichten, um den gequetschten Wirbelkörper, hierdurch die gezerrte und schmerzende Knochenhaut zu entlasten, die Zwischenwirbelgelenkfunktion zu verbessern und hierdurch dauernden Gelenkschaden zu verhindern. Dies verbessert die Behandlungsmöglichkeiten nach frischer Fraktur und bei chronischer Wirbelsäulendeformierung. Die vorliegende Erfindung erlaubt diese Aufrichtung durch Anmodellieren der gekrümmten Wirbelsäule an eine Orthese, die sich von hinten an den Rücken anlegt. Sie vermag, hierdurch Schmerzauslösung zu verhindern, die Mobilisierung zu verbessern.

Alle bisher mit diesem Behandlungsziel eingesetzten Orthesen erstreben dieses Ziel durch Einschnüren (elastische Bauchbinden) oder Druckauslösung auf Strukturen, die als Widerlager zur Aufrichtung genutzt werden. Dies engt die Patienten ein, behindert ihre Beweglichkeit in zum Teil unerträglichen Maße und führt dazu, dass diese Hilfsinstrumente nicht in wünschenswertem Umfang genutzt werden. Das mühsame Anlegen der Orthesen gemäß des Standes der Technik, sowie die genannten Einschränkungen werden hierdurch in der Regel zum Hindernis für eine angemessene Nutzung eines an sich wünschenswerten therapeutischen Ansatzes.

Die vorliegende Erfindung vermag einerseits, die Wirbelsäule wünschenswert aufzurichten, dies jedoch konstruktionsbedingt, ohne die Brust- und Bauchatmung zu behindern und ohne die Beweglichkeit im Schulter- und Armbereich einzuschränken, sowie mit einer vereinfachten und schmerzfreien Anlegemöglichkeit durch den Nutzer.

Dies steigert die Akzeptanz dieser neuen Orthese durch den Patienten und verbessert hierdurch deren Behandelbarkeit eindrucksvoll. Die Behandelbarkeit von Patienten mit Osteoporose und Wirbelkörpereinbrüchen wird optimiert.

Zusammengefasst ist festzuhalten, dass die bekannten Lösungen Brust- und Bauchatmung, sowie die Beweglichkeit im Schulter- und Armbereich einschränken. Aufgabe der vorliegenden Erfindung ist es daher, eine Minimalorthese zur Behandlung der Osteoporose zu schaffen, bei der die Brust- und Bauchatmung, sowie die Beweglichkeit im Schulter- und Armbereich möglichst wenig eingeschränkt werden.

Diese Aufgabe wird mit den im kennzeichnenden Teil des Anspruchs 1 genannten Merkmalen gelöst. Fortbildungen und vorteilhafte Ausführungen der Erfindung sind in den weiteren Ansprüchen umfasst.

Erfindungsgemäß ist eine Minimalorthese zur Behandlung der Osteoporose, bestehend aus einer die Wirbelsäule abstützenden steifen Schiene, die sich stützend im Wesentlichen über die gesamte Länge der Wirbelsäule erstreckt, sowie aus einem unteren Befestigungsgurt für die Umspannung des Rumpfes, der mit einer Abdominalpelotte, gebildet aus zwei miteinander verschließ- und lösbaren Halbteilen verbunden ist, und aus oberen Befestigungsgurten, die nach Art von Rucksackträgern über die Schultern und zurück unter den Achseln hindurch geführt sind, wobei die Brustatmung nicht behindert wird, dadurch gekennzeichnet, dass der untere Befestigungsgurt in einer Höhe angeordnet ist, in der die Bauchatmung nicht behindert wird, und dass sich die Schultergurte nach unten erstrecken, in seitlichen Umlenkschlitzen der Schiene umgelenkt werden, mit Gurtabschnitten zu der Abdominalpelotte geführt und in Befestigungsschlitzen der Abdominalpelotte befestigt sind.

Vorzugsweise erstreckt sich die Schiene in ihrer Breite über weniger als die Hälfte der Körperbreite und reicht vom Steißbein bis zum ersten Halswirbel, wodurch die Vorwärtsneigung und Beugebeweglichkeit der Wirbelsäule im Bereich der Lendenwirbelsäule (LWS) und der unteren Brustwirbelsäule (BWS) abgestützt wird, jedoch die Seitneigung und die Beweglichkeit im Schulter- und Armbereich kaum eingeschränkt werden.

Im folgenden wird die Erfindung anhand von Zeichnungen beispielhaft näher beschrieben. Dabei zeigen:
Fig. 1 die Vorderseite einer Schiene einer Minimalorthese
Fig. 2 einen vergrößerten Schnitt entlang der Linie A-A von Fig. 1
Fig. 3 eine Seitenansicht der Schiene von Fig. 1
Fig. 4 die Rückseite der Schiene von Fig. 1
Fig. 5 einen vergrößerten Schnitt entlang der Linie B-B von Fig. 4
Fig. 6 einen vergrößerten Schnitt entlang der Linie C-C von Fig. 4
Fig. 7 eine vollständige Minimalorthese aus Schiene und Befestigungsmitteln.

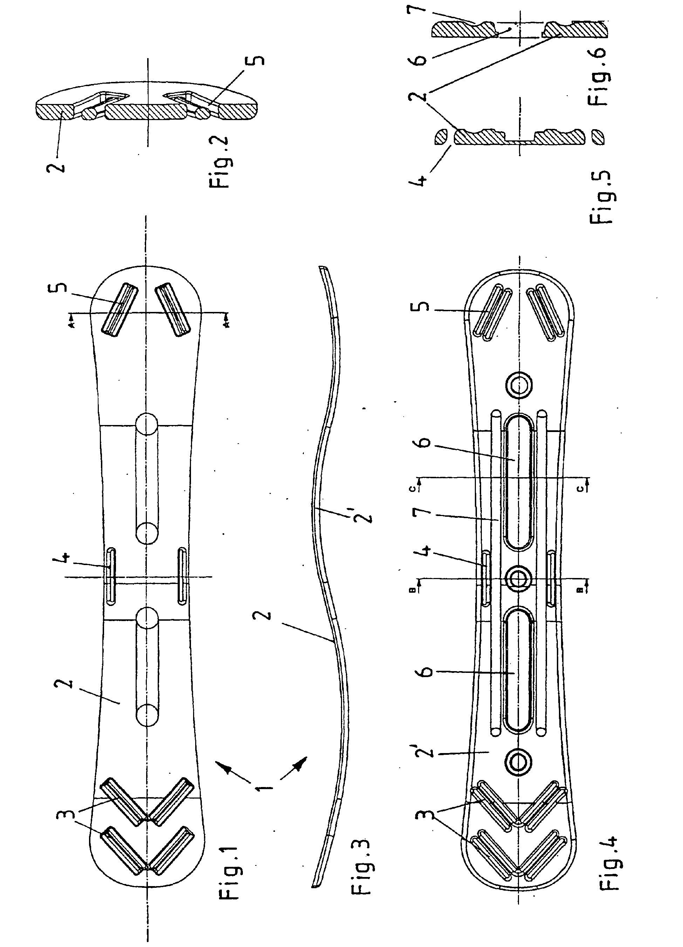

Die Schiene 1 gemäß den Fig. 1 - 6 ist ein längliches, anmodellierbares Kunststoffteil, das, wie aus Fig. 3 ersichtlich, der Wirbelsäulenanatomie entsprechend gewölbt ausgebildet ist, wobei sie sich vom Steißbein bis zum ersten Halswirbel erstreckt. Die Schiene weist obere, schräg angeordnete Befestigungsschlitze 3 für Schultergurte, mittlere, seitliche Umlenkschlitze 4 für die Schultergurte und schräg angeordnete untere Befestigungsschlitze 5 für die Befestigung von Beckengurten auf. Die Befestigungsschlitze 3 und 5 sind auf beiden Seiten jeweils paarweise entlang eines Trennsteges angeordnet, wobei die oberen Befestigungsschlitze 3 in einem Abstand voneinander doppelt vorhanden sind, um unterschiedliche Körpergrößen ausgleichen zu können. Die Schiene 1 ist aus zwei Halbteilen 2, 2' gebildet, die miteinander verschweißt sind. Zur Gewichtsverringerung sind Durchbrüche 6 und zur Erhöhung der Stabilität Längsnuten 7 vorhanden.

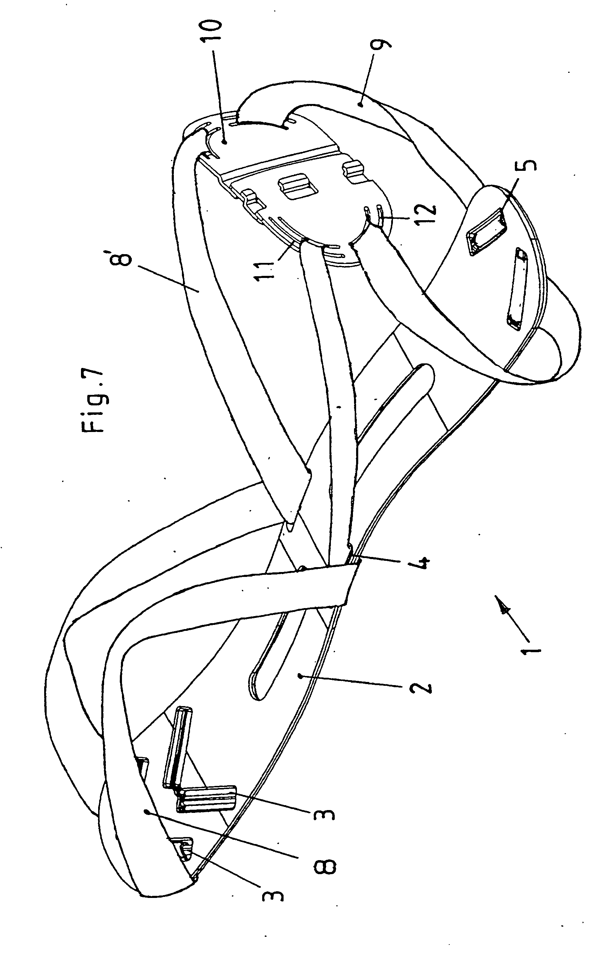

Die Befestigungsmittel sind an der Schiene 1 wie in Fig. 7 gezeigt, angeordnet. In den oberen Befestigungsschlitzen 3 ist seitlich jeweils ein Schultergurt 8 befestigt, der von der Rückseite der Schiene um diese nach vorne geführt ist. In der Art von Rucksackträgern erstrecken sich die Schultergurte 8 nach unten und werden in den seitlichen Umlenkschlitzen 4 umgelenkt und sind danach mit einem unteren Gurtabschnitt 8' zu einer Abdominalpelotte 10 geführt, an der sie in oberen Befestigungsschlitzen 11 befestigt sind. Die Abdominalpelotte 10, die aus zwei miteinander verschließ- und lösbaren Halbteilen besteht, weist weiter untere Befestigungsschlitze 12 für die Befestigung von Beckengurten 9 auf, die an ihren anderen Enden in den unteren Befestigungsschlitzen 5 der Schiene befestigt sind. Die Länge der Gurte ist entsprechend den Körpermaßen regulierbar.

## Patentansprüche

1. Minimalorthese zur Behandlung der Osteoporose, bestehend aus einer die Wirbelsäule abstützenden steifen Schiene (1), die sich stützend im Wesentlichen über die gesamte Länge der Wirbelsäule erstreckt, sowie aus einem unteren Befestigungsgurt (9) für die Umspannung des Rumpfes, der mit einer Abdominalpelotte (10), gebildet aus zwei miteinander verschließ- und lösbaren Halbteilen verbunden ist, und aus oberen Befestigungsgurten (8), die nach Art von Rucksackträgern über die Schultern und zurück unter den Achseln hindurch geführt sind, wobei die Brustatmung nicht behindert wird,
**dadurch gekennzeichnet,**
**dass** der untere Befestigungsgurt in einer Höhe angeordnet ist, in der die Bauchatmung nicht behindert wird, und dass sich die Schultergurte (8) nach unten erstrecken, in seitlichen Umlenkschlitzen (4) der Schiene (1) umgelenkt werden, mit Gurtabschnitten (8') zu der Abdominalpelotte (10) geführt und in Befestigungsschlitzen (11) der Abdominalpelotte befestigt sind.

2. Minimalorthese zur Behandlung der Osteoporose nach Anspruch 1,
**dadurch gekennzeichnet,**
**dass** sich die Schiene (1) in ihrer Breite über weniger als die Hälfte der Körperbreite erstreckt.

3. Minimalorthese zur Behandlung der Osteoporose nach Anspruch 1,
**dadurch gekennzeichnet,**
**dass** sich die Schiene (1) vom Steißbein bis zum ersten Halswirbel erstreckt.

## Claims

1. Minimal orthosis for the treatment of osteoporosis, comprising a stiff brace (1) for extending in a supporting manner substantially along the entire length of a spine; the brace having a lower pelvic belt (9) which is tensed around the body; the belt being connected to abdominal padding (10), the padding comprising two halves that may be locked together or taken apart; the brace also having upper shoulder straps (8) which pass over the shoulders and back under the armpits, like rucksack straps, in a way that does not hinder thoracic respiration,
**characterised in that**
the lower pelvic belt is arranged at a height in a way that does not interfere with abdominal respiration; the shoulder straps (8) extend downwards and are reversed in the lateral reversing slots (4) of the brace (1) before being brought together with lower belt sections (8') to the abdominal padding (10) on which they are fastened in upper fastening slots (11).

2. Minimal orthosis for the treatment of osteoporosis according to Claim1,
**characterised in that**
said brace (1) extends in width over less than half the body width.

3. Minimal orthosis for the treatment of osteoporosis according to Claim 1,
**characterised in that**
said brace (1) extends from the first neck vertebra down to the coccyx.

## Revendications

1. Orthèse minimale pour le traitement de l'ostéoporose, laquelle orthèse est constituée d'une attelle rigide (1) qui soutient la colonne vertébrale et qui s'étend en appui sensiblement sur toute la longueur de la colonne vertébrale, ainsi que d'une sangle de fixation inférieure (9) qui enserre le tronc et qui est reliée à une boucle abdominale (10) formée de deux demi-pièces pouvant se verrouiller l'une à l'autre de façon amovible et de sangles de fixation supérieures (8) qui sont guidées à la manière de supports de sac à dos par-dessus les épaules et ramenées sous les aisselles sans gêner la respiration pulmonaire,
**caractérisée en ce que** la sangle de fixation inférieure est placée à une hauteur où la respiration ventrale n'est pas gênée, et **en ce que** les sangles d'épaule (8) s'étendent vers le bas, sont déviées dans des fentes de déviation latérales (4) de l'attelle (1), sont guidées en tant que portions de sangle (8') vers la boucle abdominale (10), et sont fixées dans des fentes de fixation (11) de la boucle abdominale.

2. Orthèse minimale pour le traitement de l'ostéoporose selon la revendication 1, **caractérisée en ce que** l'attelle (1) s'étend dans sa largeur sur au moins la moitié de la largeur du corps.

3. Orthèse minimale pour le traitement de l'ostéoporose selon la revendication 1, **caractérisée en ce que** l'attelle (1) s'étend du coccyx à la première vertèbre cervicale.
